# EUROPEAN PATENT APPLICATION

(11) **EP 4 276 844 A1**
(43) Date of publication of application: **15.11.2023**
(21) Application number: 22750089.9
(22) Date of filing: 08.02.2022
(51) Int. Cl.: G16H 30/20, G16H 30/40, G16H 50/20, G06T 17/00, A61B 10/02, A61B 10/04, A61B 17/34, A61B 8/08

(54) **METHOD FOR GENERATING THREE-DIMENSIONAL PROSTATE PATHOLOGICAL IMAGE, AND SYSTEM THEREFOR**

(30) Priority: 08.02.2021 KR 20210017521
(71) Applicant: Deep Bio Inc., Seoul 08380 (KR)
(72) Inventor: KIM, Sun Woo, Seongnam-si, Gyeonggi-do 13599 (KR)
(74) Representative: Flügel Preissner Schober Seidel
(86) International application number: PCT/KR2022/001884
(87) International publication number: WO 2022/169340

(57) **Abstract**

Disclosed is a method for generating a three-dimensional prostate pathological image, and a system therefor. The method for generating a three-dimensional prostate pathological image comprises the steps of: by a system for generating a three-dimensional prostate pathological image, specifying a three-dimensional prostate image; by the system for generating a three-dimensional prostate pathological image, obtaining, through a diagnosis system, a digital diagnosis result for each of at least one specimen corresponding to predetermined template coordinates obtained through a transperineal template prostate biopsy (TTPB); and by the system for generating a three-dimensional prostate pathological image, displaying, on the three-dimensional prostate image, an onset site of prostate cancer existing in the at least one specimen on the basis of the template coordinates for each specimen and the digital diagnosis result.

## Description

### Technical Field

The present disclosure relates to a three-dimensional prostate pathological image generating system and a system thereof, and more specifically, to a method and system capable of generating a three-dimensional prostate pathology image by mapping a diagnosis result through a biopsy to a three-dimensional prostate image.

### Background Art

A transrectal ultrasound guided prostate biopsy is most commonly used as a biopsy for diagnosis of prostate cancer.

The transrectal ultrasound guided prostate biopsy is a method of collecting specimens by injecting an ultrasound probe into the rectum to see the shape of the prostate and inserting a specimen collecting needle specimens in the area where the prognosis of cancer is predicted.

However, the transrectal ultrasound guided prostate biopsy has a disadvantage in that it is not possible to accurately specify which location in the prostate the collected specimen was at, so that the vicinity of the collected specimen site must be resected relatively extensively.

In addition, recently, when performing a biopsy of the prostate, attempts have been made to increase the accuracy of the biopsy by using multiparametric magnetic response imaging (mpMRI), but even in this case, only the approximate site of the cancer may be identified, and there is a problem in that it is difficult to specify which site the collected specimen was and map it on a three-dimensional image.

Meanwhile, an examination method for relatively accurately determining the location of the collected specimen has emerged.

A transperineal template prostate mapping biopsy is an examination method in which the location of the needle collecting the specimen may be specified using a tool called a template.

Such transperineal template prostate mapping biopsy method may relatively accurately specify the location where a needle is injected through an instrument called a template, but it is only limited to specifying a location where the needle is injected.

Therefore, there is an urgent need for a method and system that may help in treatment by visualizing the location of the needle on the three-dimensional image of the prostate, the location of the collected specimen on the prostate and the location of prostate cancer expression as a result of examination of the specimen on a three-dimensional prostate image.

### [Prior Art Document]

### - Non-Patent Document

(Non-Patent Document 1) Internet publicized document:
(Non-Patent Document 2) (https://www.researchgate.net/publication/273324840_PROMIS_-_Prostate_MR_imaging_study_A_paired_validating_cohort_study_evaluating_the_role_of_ multi-parametric_MRI_in_men_with_clinical_suspicion_of_prostate_cancer)

### Disclosure of the Invention

### Technical Goals

A technical object to be achieved by the present disclosure is to provide a method and system that may be helpful in treatment by relatively accurately visualizing the location of a specimen collected through biopsy on a three-dimensional image of the prostate and the location of prostate cancer expression as a result of examination of the specimen.

### Technical Solutions

A method of generating a three-dimensional prostate pathological image for achieving the above technical objects includes specifying, by a three-dimensional prostate pathological image generating system, a three-dimensional prostate image, obtaining, by the three-dimensional prostate pathological image generating system, a digital diagnosis result for each of at least one specimen corresponding to predetermined template coordinates obtained through transperineal template prostate biopsy (TTPB) through a diagnosis system, and displaying, by the three-dimensional prostate pathological image generating system, an onset site of prostate cancer present in the at least one specimen on the three-dimensional prostate image based on template coordinates for each specimen and the digital diagnosis result.

The displaying, by the three-dimensional prostate pathological image generating system, of the onset site of prostate cancer present in the at least one specimen on the three-dimensional prostate image based on the template coordinates for each specimen and the digital diagnosis result may include, specifying, by the three-dimensional prostate pathological image generating system, a specimen location for each specimen on the three-dimensional prostate image based on the template coordinates for each specimen, and specifying, for each specimen, a relative onset location within the specimen of the prostate cancer onset site present in the specimen based on the digital diagnosis result.

The method of generating the three-dimensional prostate pathological image may further include displaying the specimen location for each specimen on the three-dimensional prostate image.

The method of generating the three-dimensional prostate pathological image may further include specifying an estimated onset site based on a plurality of onset sites respectively included in a plurality of different specimens, and displaying the estimated onset site on the three-dimensional prostate image.

The specifying of the estimated onset site based on the plurality of onset sites respectively included in the plurality of different specimens may include, when it is determined that a first individual onset site and a second individual onset site corresponding to a first specimen and a second specimen, respectively, among the plurality of different specimens satisfy a predetermined combining criterion, specifying a tissue between the first individual onset site and the second individual onset site as the estimated onset site.

The combining criterion may be a case where it is determined that the first individual onset site and the second individual onset site corresponding to the first specimen and the second specimen, respectively, satisfy an adjacency criterion according to a predetermined criterion or the first individual onset site and the second individual onset site satisfy a predetermined continuity criterion.

The specifying, for each specimen, of the relative onset location within the specimen of the prostate cancer onset site present in the specimen based on the digital diagnosis result may include, specifying the relative onset location within the specimen based on a specimen pathological image of the specimen displayed in the digital diagnosis result and a location of the prostate cancer onset site within the specimen displayed on the specimen pathological image.

The specifying, by the three-dimensional prostate pathological image generating system, of the specimen location for each specimen on the three-dimensional prostate image based on the template coordinates for each specimen may include, based on a location of a needle on an ultrasound image obtained during the TTPB, specifying a location of the needle on the three-dimensional prostate image, and specifying the specimen location for each specimen based on the location of the needle on the three-dimensional prostate image and a location of a predetermined specimen collecting part on the needle.

The method may be implemented by a computer program stored in a non-transitory computer readable recording medium.

According to another aspect of the present disclosure, a system for generating a three-dimensional prostate pathological image includes a processor, and a memory in which a program executed by the processor is recorded, wherein the processor is configured to drive the program to specify a three-dimensional prostate image, obtain a digital diagnosis result for each of at least one specimen corresponding to predetermined template coordinates obtained through transperineal template prostate biopsy (TTPB) through a diagnosis system, and display an onset site of prostate cancer present in the at least one specimen on the three-dimensional prostate image based on template coordinates for each specimen and the digital diagnosis result.

The processor may be configured to drive the program to specify a specimen location for each specimen on the three-dimensional prostate image based on the template coordinates for each specimen, and specify a relative onset location within the specimen of the prostate cancer onset site present in the specimen for each specimen based on the digital diagnosis result.

The processor may be configured to drive the program to specify an estimated onset site based on a plurality of onset sites respectively included in a plurality of different specimens, and display the estimated onset site on the three-dimensional prostate image.

The processor may be configured to drive the program to, when it is determined that a first individual onset site and a second individual onset site corresponding to a first specimen and a second specimen, respectively, among the plurality of different specimens satisfy a predetermined combining criterion, specify a tissue between the first individual onset site and the second individual onset site as the estimated onset site.

The combining criterion may be a case where it is determined that the first individual onset site and the second individual onset site corresponding to the first specimen and the second specimen, respectively, satisfy an adjacency criterion according to a predetermined criterion or the first individual onset site and the second individual onset site satisfy a predetermined continuity criterion.

The processor may be configured to drive the program to specify the relative onset location within the specimen based on a specimen pathological image of the specimen displayed in the digital diagnosis result and a location of the prostate cancer onset site within the specimen displayed on the specimen pathological image.

The processor may be configured to drive the program to, based on a location of a needle on an ultrasound image obtained during the TTPB, specify a location of the needle on the three-dimensional prostate image, and specify the specimen location for each specimen based on the location of the needle on the three-dimensional prostate image and a location of a predetermined specimen collecting part on the needle.

### Advantageous Effects

According to the technical idea of the present disclosure, by relatively accurately visualizing the location of a specimen collected through a biopsy and the location of prostate cancer expression as a result of examination of the specimen on a three-dimensional image of the prostate, there is an effect of usefully using for diagnosis, treatment, and prognosis.

### Brief Description of Drawings

In order to more fully understand the drawings cited in the detailed description of the present disclosure, a brief description of each drawing is provided.
FIG. 1 is a diagram for illustrating a concept of transperineal template prostate biopsy.
FIG. 2 is a diagram for illustrating a concept of transperineal template prostate biopsy in more detail.
FIG. 3 illustrates a schematic system configuration for implementing a three-dimensional prostate pathological image generating method in accordance with an embodiment of the present disclosure.
FIG. 4 illustrates a schematic system configuration for implementing a three-dimensional prostate pathological image generating system in accordance with an embodiment of the present disclosure.
FIG. 5 is a diagram illustrating a three-dimensional prostate image in accordance with an embodiment of the present disclosure.
FIG. 6 is a diagram for illustrating a method of expressing a location of a specimen and a cancer onset site on a three-dimensional prostate image in accordance with an embodiment of the present disclosure.
FIG. 7 is a diagram illustrating locations of a plurality of specimens on a three-dimensional prostate image in accordance with an embodiment of the present disclosure.
FIG. 8 is a diagram for illustrating an estimated onset site in accordance with an embodiment of the present disclosure.
FIG. 9 is a diagram for illustrating a combined onset site in accordance with an embodiment of the present disclosure.

### Best Mode for Carrying Out the Invention

In order to fully understand the present disclosure and the advantages in operation of the present disclosure and the objects achieved by the practice of the present disclosure, reference should be made to the accompanying drawings illustrating preferred embodiments of the present disclosure and the contents described in the accompanying drawings.

In addition, in the present specification, when one component 'transmits' data to another component, it means that the component may directly transmit the data to the other component, or may transmit the data to the other component through at least one other component. Conversely, when one component 'directly transmits' data to another component, it means that the data is transmitted from the component to the other component without going through the other component.

Hereinafter, the present disclosure will be described in detail by describing preferred embodiments of the present disclosure with reference to the accompanying drawings. Like reference numerals in each drawing indicate like elements.

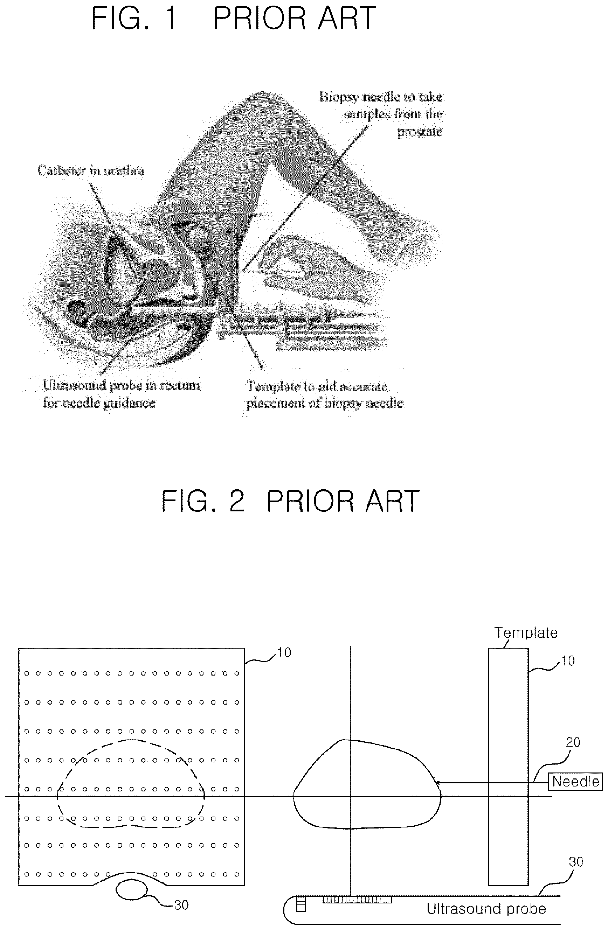

FIG. 1 is a diagram for illustrating a concept of transperineal template prostate biopsy.

In addition, FIG. 2 is a diagram for illustrating a concept of transperineal template prostate biopsy in more detail.

First, while FIG. 1 is illustrating a conventionally known transperineal template prostate biopsy method, as shown in FIG. 1, transperineal template prostate biopsy is different from the method in which a needle is injected through the rectum in the conventional transrectal prostate biopsy and the location of the needle is arbitrarily determined by the specimen collector in that it is a method of injecting a needle through the perineum after relatively accurately fixing the location of the needle through a fixing instrument called a template.

Then, while the injection location of the needle is fixed through the template, the needle is injected while checking one section of the prostate through an ultrasound probe injected through the rectum, the needle is injected into a site suspected of having prostate cancer, and a specimen of a predetermined size is collected.

In addition, referring to FIG. 2, the diagram shown on the left shows a view when an examiner (a person who collects a specimen) looks at the template 10 from the front, and the diagram shown on the right is a view illustratively showing a side view in which a needle is injected from the examiner's side.

As shown in FIG. 2, a plurality of injection ports are fixedly formed in the template 10, and identifiers capable of identifying the plurality of injection ports may be assigned.

In the present specification, the identifiers of each injection port are defined as template coordinates, and the left, right, top, and bottom locations in the front view of the prostate where the needle is injected, i.e., the locations on the x and y planes, may be specified by these template coordinates.

The number of injection ports formed on the template 10 and at what intervals may vary according to embodiments.

The examiner may select a template coordinate suitable for a part where it is determined that a specimen needs to be collected through an ultrasound image obtained through an ultrasound probe 20, and inject the specimen collecting needle 20 into the selected template coordinate.

Through this, there is an effect of relatively accurately specifying the location of the needle 20 from which the specimen was collected, compared to the conventional transrectal prostate biopsy method.

However, in such transperineal template prostate biopsy method, although the x, y coordinates are specified in the front view (e.g., the left diagram in FIG. 2) of the template 10, not only there is a problem in that it is difficult to specify which part the specimen collected from the front view is in the depth direction of the prostate (e.g., left and right direction in the right diagram of FIG. 2), but also, even if the depth direction may be known, when cancer is expressed in a specific site of the specimen as a result of diagnosis of the specimen collected, there is a problem in that it cannot be known what location the specific site is in the entire prostate.

Therefore, according to the technical idea of the present disclosure, while examining through the above-mentioned transperineal template prostate biopsy, if cancer is expressed in the specimen collected and a specific site in the specimen, it is possible to provide a method and system capable of visually and easily confirming examination results by relatively accurately mapping the expression location of the cancer on a three-dimensional prostate image and also usefully utilized for treatment.

In a method of generating a three-dimensional prostate pathological image according to the technical idea of the present disclosure, when one or more specimens were collected from the prostate via transperineal template prostate biopsy to perform examination, in addition to simply confirming whether or not cancer is expressed in the specimen, it is possible to specify a site in the specimen in which cancer is expressed and display it on a three-dimensional prostate image.

In particular, when a specimen is collected over a certain length in the longitudinal direction of the needle and cancer is expressed only in a certain site of the specimen, only the certain site in the specimen is displayed on the three-dimensional prostate image, thereby enabling relatively accurate visual confirmation and treatment of examination results, and may be effective in preventing treatment for excessive sites.

Moreover, according to the technical idea of the present disclosure, it is possible to provide an effect of easily confirming the examined site on the prostate image by displaying the location of the collected specimen as well as displaying the cancer expression site on the three-dimensional prostate image.

In addition, since it is possible to relatively accurately determine the location of cancer expression even within a specimen, in case of collecting a plurality specimens, based on the location of cancer expression in each of the plurality of specimens, it is possible to provide an effect that may reasonably predict and visualize a location where cancer is highly likely to be expressed even though the specimen has not actually been collected.

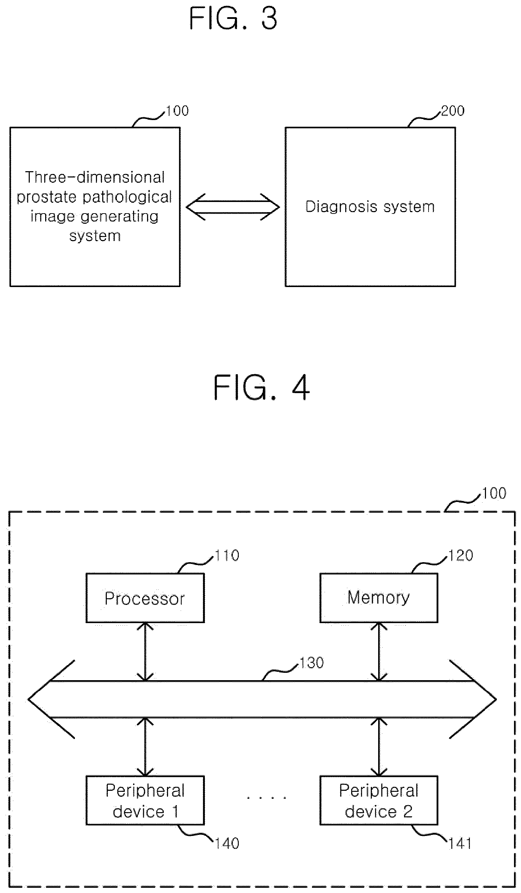

A three-dimensional prostate pathological image generating system according to the technical idea of the present disclosure for implementing this technical idea will be described with reference to FIGS. 3 and 4.

FIG. 3 illustrates a schematic system configuration for implementing a three-dimensional prostate pathological image generating method in accordance with an embodiment of the present disclosure. In addition, FIG. 4 illustrates a schematic system configuration for implementing a three-dimensional prostate pathological image generating system in accordance with an embodiment of the present disclosure.

Referring to FIG. 3, a three-dimensional prostate pathological image generating system 100 may be provided in order to implement a method of generating a three-dimensional prostate pathological image according to the technical idea of the present disclosure.

The three-dimensional prostate pathological image generating system (hereinafter referred to as 'generating system', 100) may implement the technical idea of the present disclosure while communicating with a predetermined diagnosis system 200.

In addition, the generating system 100 may receive the examination result of the specimen from the diagnosis system 200, and based on the examination result, may map and display the location of the specimen and the expression location of cancer on the three-dimensional prostate image as described above.

The diagnosis system 200 may be a system that receives a pathological image (e.g., a tissue image) of a specimen and determines in which site prostate cancer is expressed.

The diagnosis system 200 may be a system that determines the expression part of cancer on the entire digital image of the specimen, i,e., on a slide image or a patch image obtained by dividing the slide image into a plurality of patches through deep learning-based image analysis to display the location of the expression site on the image or output the location of the expression site of the cancer.

An example of such a diagnosis system 200 has been disclosed in Korean patent applications (Application No. 10-2016-0168176, System and Method for Medical Diagnosis Using Neural Network), (Application No. 10-2018-0064332, System and Method for Two Phase Diagnosis Using Neural Network) filed by the present applicant, and in addition, various diagnosis methods are known that receive a digital image of a tissue and specify a cancer onset site through a deep learning-based diagnosis model.

Although in the present specification, a detailed description of the diagnosis system 200 itself is omitted to clarify the gist of the present disclosure, an average expert in the art of the present disclosure will be able to easily infer that various methods of receiving a digital image of a specimen to be examined and specifying the onset site of prostate cancer on the digital image may be implemented with the diagnosis system 200.

The diagnosis system 200 may receive an input of the injection port of the specimen collected by the examiner, i.e., the coordinates of the template, from the examiner, and the diagnosis system 200 may transfer template coordinates and digital diagnosis results of a specimen corresponding to the template coordinates to the generating system 100.

In addition, the diagnosis system 200 may be provided with a tool for generating a digital image of the specimen by photographing or scanning the collected specimen when the specimen is collected, or may receive a digital image through the tool.

The digital diagnosis result of the specimen may be a pathological image itself in which an onset site of prostate cancer is marked on a digital image corresponding to the specimen. In this case, the generating system 100 may analyze the pathological image to specify the location (e.g., coordinate values, etc.) of the cancer onset site. Alternatively, according to an embodiment, the diagnosis system 200 may specify at least one coordinate value that may indicate the prostate cancer expression site on the digital image of the specimen and transmit to the generating system 100. These coordinate values may be used as relative locations indicating the onset site of prostate cancer in the corresponding specimen.

According to an embodiment, information (e.g., Gleason score) representing the progress level of prostate cancer by site of expression may be transmitted from the diagnosis system 200 to the generating system 100, and the generating system 100 may visualize the corresponding site differentially according to the progress level of prostate cancer as well as the location of the cancer expression site on the three-dimensional prostate image.

Then, the generating system 100 may receive template coordinates for each specimen and a digital diagnosis result corresponding to the specimen, and may specify a location of the corresponding specimen on the three-dimensional prostate image based on the received template coordinates for each specimen.

As for the location of the specimen on the three-dimensional prostate image, the locations of x and y on the front view of the template 10 may be specified based on the template coordinates for each specimen. In addition, the location in the depth direction may be estimated based on the length of the needle and the location of the collecting part formed on the needle to collect the specimen.

Depending on embodiments, the ultrasound image in a state in which the needle is injected may be analyzed in order to calculate a more accurate depth direction. For example, the generating system 100 may additionally receive an ultrasound image in a state in which the needle is injected, and may specify the location of the outer edge of the prostate and the end of the needle in the ultrasound image through image analysis.

Then, by converting the location of the needle analyzed in the ultrasound image into a location on the three-dimensional prostate image, the location of the injected needle in the depth direction may be determined relatively accurately, and when the location of the needle is specified, the location of the specimen collected on the three-dimensional prostate image may be finally specified based on the location of the specimen collecting part formed at a predetermined location in the needle.

In addition, the generating system 100 may confirm information about the relative location of the prostate cancer onset site and/or the progress level of cancer based on the digital diagnosis result of the diagnosis system 200.

Then, based on the location of the specified specimen and the relative onset location of the cancer onset site (onset site) within the specimen, the cancer onset site in the corresponding specimen may finally be specified and displayed on the three-dimensional prostate image.

In addition, by performing this process on all specimens collected, the diagnosis results diagnosed through transperineal template prostate biopsy may be visualized on the three-dimensional prostate image.

In addition, according to the technical idea of the present disclosure, when a plurality of specimens are collected and each specimen has an onset site of cancer, based on the location of the specimen and the location of the onset site of cancer for each specimen, it is possible to estimate whether or not cancer has developed in the prostate site that has not actually been collected. In other words, an estimated onset site may be calculated and provided.

A specific configuration of the generating system 100 for providing such a technical idea may be as shown in FIG. 4.

As shown in FIG. 2, the generating system 100 includes a processor 110 and a storage medium (or memory, 120) for implementing the functions defined in the present specification. The processor 110 may refer to an arithmetic device capable of executing a predetermined program (software code) and may be named by various names such as an embodiment of the data processing device or a vendor mobile processor, microprocessor, CPU, single processor, multiprocessor, GPU, etc., and may be implemented as one or more processors.

The average expert in the technical field of the present disclosure may easily infer that the processor 110 may perform data processing necessary for the technical idea of the present disclosure by driving the above program.

The memory 120 may mean a device in which a program for implementing the technical idea of the present disclosure is stored/installed. According to an embodiment, the memory 120 may be divided into a plurality of different physical devices, and a part of the memory 120 may exist inside the processor 110 according to an embodiment. The memory 120 may be implemented as a hard disk, a GPU, a solid state disk (SSD), an optical disk, a random access memory (RAM), and/or other various types of storage media, depending on an embodiment, and may be detachably implemented in the memory 120 if neccessary.

The generating system 100 may be implemented as an independent physical device for implementing a method of generating a three-dimensional prostate pathological image according to the technical idea of the present disclosure while communicating with the diagnosis system 200, but is not limited thereto, and may be implemented with any data processing device (e.g., computer, mobile terminal, etc.) capable of processing data to execute the program.

According to an embodiment, the generating system 100 may be mounted on the diagnosis system 200 and physically implemented integrally with the diagnosis system 200.

In addition, the average expert in the technical field of the present disclosure may easily infer that the generating system 100 may be provided with the processor 110, the memory 120, and various peripheral devices (e.g., input/output devices, display devices, audio devices, etc., 140, 141) provided in the diagnosis control system 100, and a communication interface (e.g., communication bus, 130, etc.) to connect these devices.

Meanwhile, the generating system 100 according to the technical idea of the present disclosure may be implemented by organically combining the program (or software) stored in the memory 120 and the processor 110, and the average expert in the technical field of the present disclosure may easily infer that the function and/or operation performed by the generating system 100 in the present specification hereinafter may be executed by the processor 110.

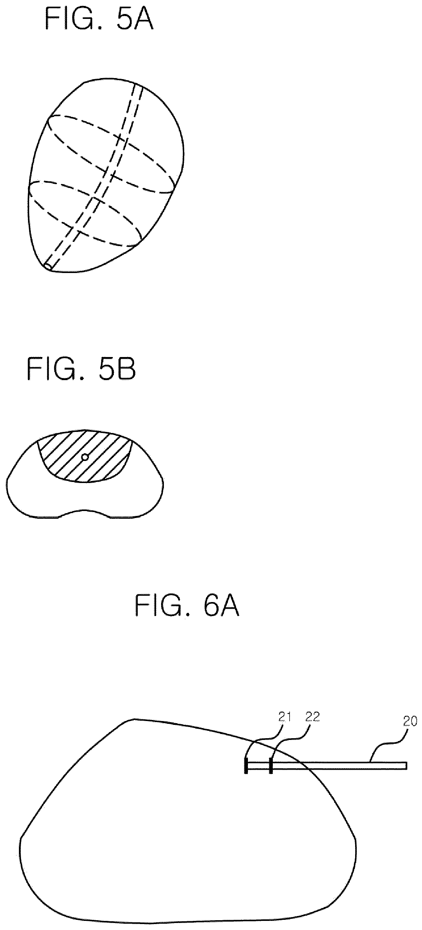

FIG. 5 is a diagram illustrating a three-dimensional prostate image in accordance with an embodiment of the present disclosure.

FIGS. 5A and 5B illustrate a three-dimensional prostate image and a front view of a template 10, respectively, and the generating system 100 may specify such three-dimensional prostate image and visualize a cross section in a desired direction as needed.

The three-dimensional prostate image may be received from the outside by the generating system 100 or may be generated by the generating system 100 itself.

The technical idea for generating three-dimensional prostate images is widely known. For example, a three-dimensional image of the subject's prostate may be generated through techniques such as CT 3D and/or multiparametric magnetic resonance image (mpMRI).

The generating system 100 may display a schematic, i.e., an estimated cancer expression site on the generated three-dimensional image using information acquired through a predetermined diagnosis method (e.g., ultrasound scan, mpMRI, etc.).

The examiner may perform the transperineal template prostate biopsy as described above at the expected site of expression.

A specimen collected through the transperineal template prostate biopsy may be diagnosed through the diagnosis system 200.

The diagnosis system 200 may determine the onset site of prostate cancer in the specimen for each specimen and transmit the digital diagnosis result to the generating system 100. In this case, template coordinates of each specimen may be input to the diagnosis system 200.

The generating system 100 may specify and visualize the location of the specimen and/or the onset site of cancer on the three-dimensional prostate image based on the received diagnosis result for each specimen.

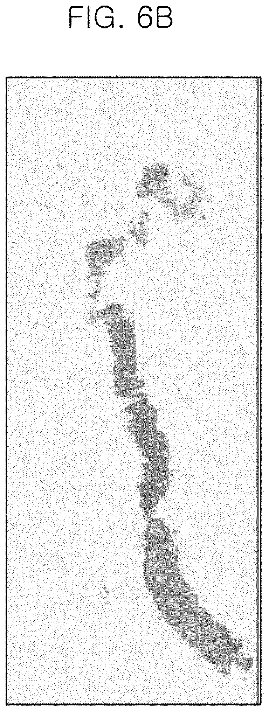

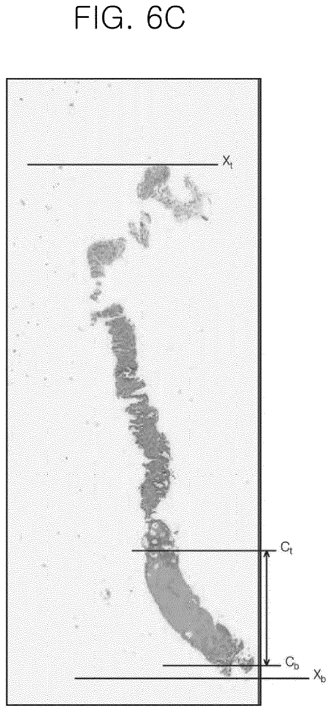

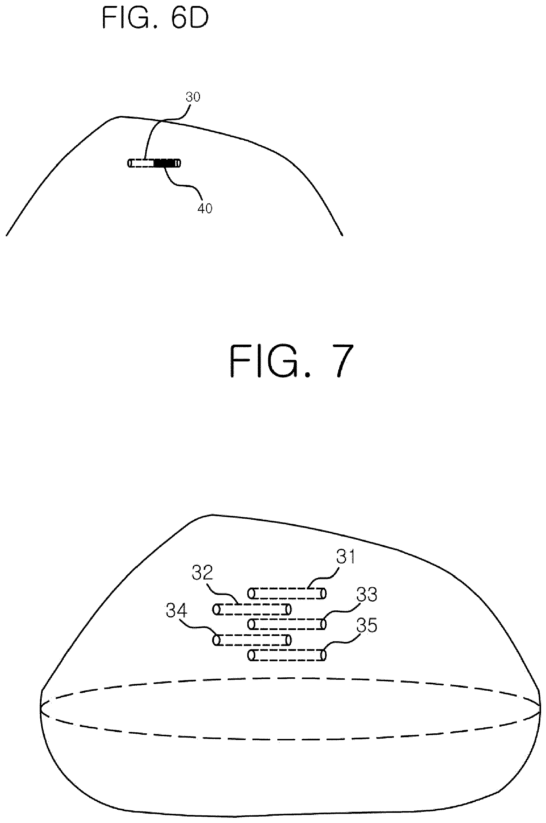

FIG. 6 is a diagram for illustrating a method of expressing a location of a specimen and a cancer onset site on a three-dimensional prostate image in accordance with an embodiment of the present disclosure.

First, referring to FIG. 6A, FIG. 6A illustrates a view in the case of performing a transperineal template prostate biopsy through the needle 20 from the side with respect to the template 10, and in a state in which a predetermined coordinate of the template is selected, the needle 20 may be inserted into the injection port corresponding to the coordinate of the template, and the needle 20 may reach a predetermined location of the prostate.

Then, the specimen collecting part formed at a predetermined location (e.g., 21 to 22) of the needle 20 may collect a specimen by a certain length in the longitudinal direction of the needle.

The collected specimen may be converted into a digital pathological image by the diagnosis system 200 or through a separate external device.

The diagnosis system 200, receiving the converted digital pathological image and template coordinates, may generate a digital diagnosis result.

An example of the generated digital diagnosis result may be as shown in FIG. 6B.

FIG. 6B exemplarily illustrates a case in which the onset site of prostate cancer is visualized in yellow and orange in the digital pathological image of the specimen of the part marked in pink.

At this time, the areas visualized in yellow and orange may be visualized in different colors to distinguish different progress levels of prostate cancer.

The digital diagnosis result may be the image itself in which the onset site of prostate cancer is visualized in the digital pathological image of the specimen, or may be a coordinate value for each pixel corresponding to the onset site of prostate cancer on the digital pathological image and a progress level (e.g., Gleason score) corresponding to each coordinate value.

In any case, the digital diagnosis result may include information capable of specifying which location on the digital pathological image of the specimen is the onset site of prostate cancer.

Then, as shown in FIG. 6C, the generating system 100 may specify the relative onset location (e.g., Ct to Cb) of the prostate cancer within the entire length (e.g., Xt to Xb) of the specimen.

The relative onset location in the specimen may be a start location (Ct) and an end location (Cb) that may specify the location of onset of prostate cancer in the specimen as shown in FIG. 6C, or the coordinate values of pixels corresponding to the onset site in the case of using the specimen as a coordinate system may be the relative onset location.

In any case, when the location of the specimen is specified on the three-dimensional prostate image, any information that may specify the location of the onset site in the specimen on the three-dimensional prostate image may be the relative onset location.

In addition, as described above, the location of the specimen may be specified according to the template coordinates, the location of the needle 20 in the depth direction, and the location of the specimen collecting part in the needle.

In addition, while the location of the needle 20 in the depth direction may be calculated as a rough estimate based on how much the needle has entered with respect to the template 10, as described above the location of the needle 20 may be determined by the generating system 100 through an ultrasound image obtained when specimen collection is performed.

For example, as described above, the location of the needle 20 in the depth direction on the three-dimensional image may be determined in more detail by analyzing the distance difference between the outer edge of the prostate and the tip of the needle 20 in the ultrasound image.

Then, as shown in FIG. 6D, the onset site 40 of prostate cancer may be specified and visualized on the three-dimensional prostate image.

The generating system 100 may simply visualize only the onset site of prostate cancer on the three-dimensional prostate image, but since it may be important information for diagnosis and treatment, the location of the specimen 30 collected may be displayed on the three-dimensional prostate image.

In addition, while FIG. 6 shows a case where the location of the specimen 30 and/or the onset site 4 of prostate cancer is visualized for any one specimen, in general, since a plurality of specimens are collected at a plurality of locations, this case may be as shown in FIG. 7.

FIG. 7 is a diagram illustrating locations of a plurality of specimens on a three-dimensional prostate image in accordance with an embodiment of the present disclosure.

Referring to FIG. 7, for example, through transperineal template prostate biopsy, five specimens may be collected at locations corresponding to different template coordinates, and in this case, when the locations of the specimens 31, 32, 33, 34, and 35 are visualized on the three-dimensional prostate image, they may be as shown in FIG. 7.

In addition, the generating system 100 may specify the onset site of prostate cancer for each specimen and visualize it.

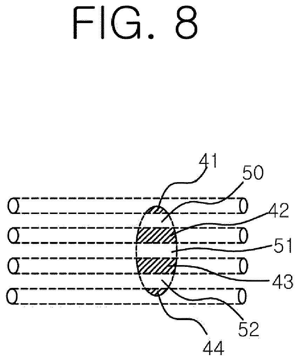

FIG. 8 is a diagram for illustrating an estimated onset site in accordance with an embodiment of the present disclosure.

FIG. 8 simplifies and illustrates a case in which when a plurality of specimens are collected, individual onset sites 41, 42, 43, and 44 in respective specimens are visualized on the three-dimensional prostate image by the generating system 100.

According to the technical idea of the present disclosure, the generating system 100 may stop at simply visualizing the onset sites 41, 42, 43, and 44 for each of the plurality of specimens on the three-dimensional prostate image.

However, according to the technical idea of the present disclosure, based on the plurality of onset sites 41, 42, 43, and 44, estimated onset sites (e.g., 50, 51, and 52) may be specified, and these estimated onset sites (e.g., 50, 51, and 52) may be further visualized.

This is because prostate cancer may occur even in sites that are not collected through transperineal template prostate biopsy and when there are onset sites 41, 42, 43, and 44 that satisfy predetermined reference conditions, there is a high possibility that prostate cancer has occurred at a predetermined site, i.e., an estimated onset site, that has not been collected, and it may be very useful to visualize even these estimated onset sites.

In other words, the generating system 100 may visualize the estimated onset site separately from the onset site by actual diagnosis, or display and visualize combined onset sites including the estimated onset sites 50, 51, and 52 and the actual onset sites 41, 42, 43, and 44 on the three-dimensional prostate image.

The estimated onset site may be a site that satisfies a predetermined reference condition, i,e., a combining criterion.

For example, when it is determined that the first individual onset site (e.g., 41) and the second individual onset site (e.g., 42) corresponding to the first specimen and the second specimen, respectively, among the plurality of different specimens satisfy the predetermined combining criterion, the generating system 100 may specify the tissue (e.g., 50) between the first individual onset site (e.g., 41) and the second individual onset site (e.g., 42) as the estimated onset site.

Alternatively, when it is determined that the first individual onset site (e.g., 42) and the second individual onset site (e.g., 43) satisfy the predetermined combining criterion, the tissue (e.g., 51) between the first individual onset site (e.g., 42) and the second individual onset site (e.g., 43) may be specified as the estimated onset site.

The combining criterion may include a condition that the template coordinates corresponding to each of the first specimen and the second specimen satisfy an adjacency criterion determined by a predetermined criterion.

In other words, when the locations of individual onset sites are adjacent to each other at a certain level or more, there is a high probability of occurrence in tissues between individual onset sites, so such condition may be included in the above combining criterion.

Whether the locations of individual onset sites are adjacent may be simply determined based on the template coordinates of the specimens corresponding to the individual onset sites, or may be determined based on the distance between actual individual onset sites in three dimensions.

The distance between individual onset sites in three dimensions may be the distance between center points of the individual onset sites, or may be the distance between the nearest outlines after specifying the outline of each of the individual onset sites. The distance between individual onset sites may be defined in various ways, and the degree of adjacency to satisfy the combining criterion may be determined in advance or determined through repeated experiments.

In addition, since it may not be considered that the tissue between the individual onset sites is diseased simply because they are adjacent to each other, whether or not a predetermined continuity criterion between the individual onset sites is satisfied may be further included in the combining criterion.

The continuity criterion may be, for example, a case in which the progression of the disease is the same or with a difference within a predetermined range, such as that the Gleason scores of two individual onset sites satisfying the adjacency criterion are the same or only have a difference of about 1. Alternatively, it may be a case where individual onset sites satisfy a predetermined pattern shape. This pattern shape is learned from a number of medical data to build a pattern for the shapes of the onset site in advance, and it may be determined that the continuity criterion is satisfied when at least one of such pattern shapes may include two individual onset sites.

The continuity criterion may be defined in a variety of ways.

In general, when there are two separate onset sites that satisfy the adjecency criterion, resection or treatment is also performed in the area between them, so the continuity criterion may not need to be defined too strictly.

In addition, the combining criterion may be defined to be satisfied even if only one of the adjacency criterion or the continuity criterion is satisfied, or the combining criterion may be defined to be satisfied only when both of the criterion are satisfied.

After all, according to the technical idea of the present disclosure, it is not limited to the onset site confirmed through actual diagnosis being visualized on a three-dimensional prostate image, but it may be very useful for diagnosis and treatment by estimating and visualizing even sites with a high possibility of onset based on the confirmed onset site.

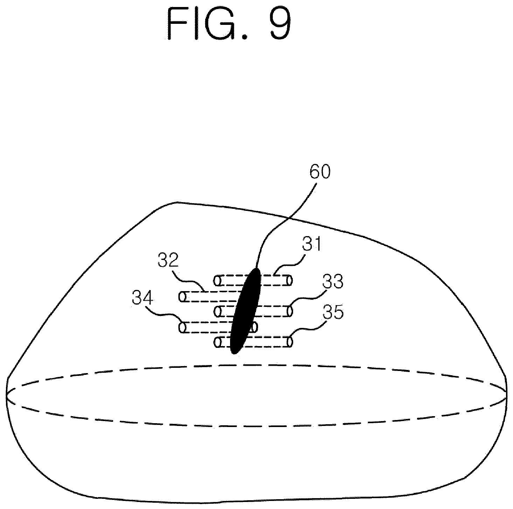

FIG. 9 is a diagram for illustrating a combined onset site in accordance with an embodiment of the present disclosure.

FIG. 9 illustrates an example in which a combined onset site including an estimated onset site and an actual onset site is displayed on a three-dimensional prostate image according to the technical idea of the present disclosure.

While FIG. 9 exemplarily shows a case where the estimated onset site and the actual onset site are visualized without distinction so that the location and size of the entire onset site may be easily visualized, as described above, the generating system 100 may also visualize so that the actual onset site and the estimated onset site may be distinguished.

In addition, for convenience of explanation, in the present specification, it has been described that visualization is performed based on whether or not prostate cancer has occurred, but as described above, the onset site may be classified and visualized according to the progress level (e.g., Gleason score) of prostate cancer.

The method of generating a three-dimensional prostate pathological image according to an embodiment of the present disclosure may be implemented as computer readable code on a non-transitory computer readable recording medium. A non-transitory computer-readable recording medium includes all types of recording devices in which data that may be read by a computer system is stored. Examples of computer-readable recording media include ROM, RAM, CD-ROM, magnetic tape, hard disk, floppy disk, and optical data storage devices. In addition, the computer-readable recording medium is distributed in computer systems connected through a network, so that computer-readable codes may be stored and executed in a distributed manner. In addition, functional programs, codes, and code segments for implementing the present disclosure may be easily inferred by programmers in the art to which the present disclosure belongs.

Although the present disclosure has been described with reference to an embodiment shown in the drawings, this is merely exemplary, and those skilled in the art will understand that various modifications and equivalent other embodiments are possible therefrom. Therefore, the true technical protection scope of the present disclosure should be determined by the technical idea of the attached claims.

### Industrial Applicability

The present disclosure may be used for a method of generating a three-dimensional prostate pathological image and a system thereof.

## Claims

1. A method of generating a three-dimensional prostate pathological image, the method comprising:
specifying, by a three-dimensional prostate pathological image generating system, a three-dimensional prostate image;
obtaining, by the three-dimensional prostate pathological image generating system, a digital diagnosis result for each of at least one specimen corresponding to predetermined template coordinates obtained through transperineal template prostate biopsy (TTPB) through a diagnosis system; and
displaying, by the three-dimensional prostate pathological image generating system, an onset site of prostate cancer present in the at least one specimen on the three-dimensional prostate image based on template coordinates for each specimen and the digital diagnosis result.

2. The method of claim 1, wherein the displaying, by the three-dimensional prostate pathological image generating system, of the onset site of prostate cancer present in the at least one specimen on the three-dimensional prostate image based on the template coordinates for each specimen and the digital diagnosis result comprises:
specifying, by the three-dimensional prostate pathological image generating system, a specimen location for each specimen on the three-dimensional prostate image based on the template coordinates for each specimen; and
specifying, for each specimen, a relative onset location within the specimen of the prostate cancer onset site present in the specimen based on the digital diagnosis result.

3. The method of claim 1, further comprising:
displaying the specimen location for each specimen on the three-dimensional prostate image.

4. The method of claim 1, further comprising:
specifying an estimated onset site based on a plurality of onset sites respectively included in a plurality of different specimens; and
displaying the estimated onset site on the three-dimensional prostate image.

5. The method of claim 4, wherein the specifying of the estimated onset site based on the plurality of onset sites respectively included in the plurality of different specimens comprises, when it is determined that a first individual onset site and a second individual onset site corresponding to a first specimen and a second specimen, respectively, among the plurality of different specimens satisfy a predetermined combining criterion, specifying a tissue between the first individual onset site and the second individual onset site as the estimated onset site.

6. The method of claim 5, wherein the combining criterion is a case where it is determined that the first individual onset site and the second individual onset site corresponding to the first specimen and the second specimen, respectively, satisfy an adjacency criterion according to a predetermined criterion or the first individual onset site and the second individual onset site satisfy a predetermined continuity criterion.

7. The method of claim 2, wherein the specifying, for each specimen, of the relative onset location within the specimen of the prostate cancer onset site present in the specimen based on the digital diagnosis result comprises specifying the relative onset location within the specimen based on a specimen pathological image of the specimen displayed in the digital diagnosis result and a location of the prostate cancer onset site within the specimen displayed on the specimen pathological image.

8. The method of claim 2, wherein the specifying, by the three-dimensional prostate pathological image generating system, of the specimen location for each specimen on the three-dimensional prostate image based on the template coordinates for each specimen comprises:
based on a location of a needle on an ultrasound image obtained during the TTPB, specifying a location of the needle on the three-dimensional prostate image; and
specifying the specimen location for each specimen based on the location of the needle on the three-dimensional prostate image and a location of a predetermined specimen collecting part on the needle.

9. A computer program stored in a non-transitory computer readable recording medium installed in a data processing device and performing the method according to any one of claims 1 to 8.

10. A system for generating a three-dimensional prostate pathological image, the system comprising:
a processor; and
a memory in which a program executed by the processor is recorded,
wherein the processor is configured to drive the program to:
specify a three-dimensional prostate image, obtain a digital diagnosis result for each of at least one specimen corresponding to predetermined template coordinates obtained through transperineal template prostate biopsy (TTPB) through a diagnosis system, and display an onset site of prostate cancer present in the at least one specimen on the three-dimensional prostate image based on template coordinates for each specimen and the digital diagnosis result.

11. The system of claim 10, wherein the processor is configured to drive the program to:
specify a specimen location for each specimen on the three-dimensional prostate image based on the template coordinates for each specimen, and specify, for each specimen, a relative onset location within the specimen of the prostate cancer onset site present in the specimen based on the digital diagnosis result.

12. The system of claim 10, wherein the processor is configured to drive the program to:
specify an estimated onset site based on a plurality of onset sites respectively included in a plurality of different specimens, and display the estimated onset site on the three-dimensional prostate image.

13. The system of claim 12, wherein the processor is configured to drive the program to:
when it is determined that a first individual onset site and a second individual onset site corresponding to a first specimen and a second specimen, respectively, among the plurality of different specimens satisfy a predetermined combining criterion, specify a tissue between the first individual onset site and the second individual onset site as the estimated onset site.

14. The system of claim 13, wherein the combining criterion is a case where it is determined that the first individual onset site and the second individual onset site corresponding to the first specimen and the second specimen, respectively, satisfy an adjacency criterion according to a predetermined criterion or the first individual onset site and the second individual onset site satisfy a predetermined continuity criterion.

15. The system of claim 11, wherein the processor is configured to drive the program to:
specify the relative onset location within the specimen based on a specimen pathological image of the specimen displayed in the digital diagnosis result and a location of the prostate cancer onset site within the specimen displayed on the specimen pathological image.

16. The system of claim 11, wherein the processor is configured to drive the program to:
based on a location of a needle on an ultrasound image obtained during the TTPB, specify a location of the needle on the three-dimensional prostate image, and specify the specimen location for each specimen based on the location of the needle on the three-dimensional prostate image and a location of a predetermined specimen collecting part on the needle.
